(19) Europäisches Patentamt
European Patent Office
Office européen des brevets

(11) Veröffentlichungsnummer : 0 327 808 B1

# (12) EUROPÄISCHE PATENTSCHRIFT

(45) Veröffentlichungstag der Patentschrift : **02.05.91 Patentblatt 91/18**

(51) Int. Cl.[5] : **C07D 413/04**, C07D 413/14, C07D 417/04, A61K 31/445, A61K 31/535, A61K 31/495, A61K 31/54

(21) Anmeldenummer : **89100274.3**

(22) Anmeldetag : **09.01.89**

## (54) Substituierte 3-Aminosydnonimine, Verfahren zu ihrer Herstellung und ihre Verwendung.

Die Akte enthält technische Angaben, die nach dem Eingang der Anmeldung eingereicht wurden und die nicht in dieser Patentschrift enthalten sind.

(30) Priorität : **14.01.88 DE 3800830**

(43) Veröffentlichungstag der Anmeldung : **16.08.89 Patentblatt 89/33**

(45) Bekanntmachung des Hinweises auf die Patenterteilung : **02.05.91 Patentblatt 91/18**

(84) Benannte Vertragsstaaten : **AT BE CH DE ES FR GB GR IT LI NL SE**

(56) Entgegenhaltungen :
**EP-A- 0 276 710**
**DE-A- 1 620 501**
**FR-A- 2 271 818**

(73) Patentinhaber : **CASSELLA Aktiengesellschaft
Hanauer Landstrasse 526
W-6000 Frankfurt am Main 61 (DE)**

(72) Erfinder : **Schönafinger, Karl, Dr.
Holunderweg 8
W-8755 Alzenau (DE)**
Erfinder : **Beyerle, Rudi, Dr.
An der Pfaffenmauer 44
W-6000 Frankfurt am Main 60 (DE)**
Erfinder : **Bohn, Helmut, Dr.
Kranzbergring 11
W-6369 Schöneck 1 (DE)**

(74) Vertreter : **Urbach, Hans-Georg, Dr. et al
Hanauer Landstrasse 526
W-6000 Frankfurt am Main 61 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

**Beschreibung**

Die Erfindung betrifft pharmakologisch wirksame substituierte 3-Aminosydnonimine der allgemeinen Formel I

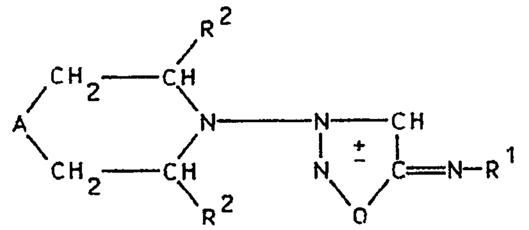

(I)

und ihre pharmakologisch annehmbaren Säureadditionssalze, worin

A den Rest $-CH_2-$, $-O-$, $-S(O_n)-$, $-N(R^3)-$ oder eine direkte Bindung,

$R^1$ Wasserstoff, Nitroso (–NO) oder den Rest $-COR^4$,

$R^2$, $R^3$ Alkyl mit 1 bis 4 C-Atomen oder Phenylalkyl mit 1 bis 4 C-Atomen im Alkylrest,

$R^4$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann ; einen cycloaliphatischen Rest mit 5 bis 7 C-Atomen ; einen bicycloaliphatischen Rest mit 7 bis 14 C-Atomen ; einen tricycloaliphatischen Rest mit 7 bis 16 C-Atomen ; einen Alkoxyrest mit 1 bis 18 C-Atomen, der auch durch Alkoxy mit 1 bis 6 C-Atomen substituiert sein kann ; einen Aryloxyrest mit 6 bis 10 C-Atomen ; einen Alkoxycarbonylrest mit insgesamt 2 bis 7 C-Atomen ; einen Arylrest mit 6 bis 10 C-Atomen ; einen Arylrest mit 6 bis 10 C-Atomen, der mono-, di- oder trisubstituiert durch 1 bis 3 Halogenatome und/oder 1 bis 3 Alkylreste mit 1 bis 4 C-Atomen und/oder 1 bis 3 Alkoxyreste mit 1 bis 4 C-Atomen und/oder 1 oder 2 Nitrogruppen und/oder 1 oder 2 Hydroxygruppen und/oder 1 oder 2 Alkylcarbonyloxyreste mit 1 bis 4 C-Atomen und/oder 1 bis 3 Alkylthioreste mit 1 bis 4 C-Atomen und/oder einen Trifluormethylrest und/oder einen Imidazolylrest ist Imidazoloyl ; Pyridyl ; Thienyl ; Styryl, n die Zahl 0, 1 oder 2, bedeuten.

Die Erfindung betrifft ferner ein Verfahren zur Herstellung der erfindungsgemäßen Verbindungen 1 und ihre Verwendung.

Falls A einen der Reste $-CH_2-$, $-O-$, $-S(O_n)-$ oder $-N(R^3)-$ bedeutet, steht in 3-Stellung des Sydnonimins der Rest eines heterocyclischen 6-Rings mit einem Heteroatom (N) oder mit zwei Heteroatomen (N,O oder N,S oder N,N), der in der angegebenen Weise substituiert ist. Falls A eine direkte Bindung bedeutet, steht in 3-Stellung des Sydnonimins ein in 2,5-Stellung disubstituierter Pyrrolidinrest.

Aliphatische Reste, Thioreste, Alkylreste, Alkoxyreste können geradkettig oder verzweigt sein. Dies gilt auch, wenn sie als Substituenten anderer Reste, z.B. als Substituenten für Arylreste, oder in Verbindung mit anderen Resten auftreten, z.B. als Phenalkyl, als Alkoxycarbonyl, als Alkylcarbonyloxy oder als Alkoxyalkoxy.

Für A sind die direkte Bindung und die zweiwertigen Reste : $-CH_2-$, $-O-$ und $-S(O_2)-$ bevorzugt, von denen der Rest $-CH_2-$ besonders bevorzugt ist.

Die für $R^2$ und $R^3$ stehenden Alkyl- oder Phenalkylreste können gleich oder verschieden sein.

Beispiele für geeignete für $R^2$ und/oder $R^3$ stehende Phenalkylreste sind Benzyl, 2-Phenethyl, 3-Phenylpropyl, 3-Phenyl-butyl. Beispiele für geeignete für $R^2$ und/oder $R^3$ stehende Alkylreste sind Methyl, Ethyl, n-Propyl, i-Propyl, n-Butyl, i-Butyl, tert.-Butyl. Für $R^2$ und/oder $R^3$ ist Methyl bevorzugt.

Als für $R^4$ stehende aliphatische Reste kommen insbesondere Alkylreste, vorzugsweise mit 1 bis 4 C-Atomen, in Betracht. Die für $R^4$ stehenden aliphatischen Reste, insbesondere Alkylreste, können auch mit Alkoxy mit 1 bis 6 C-Atomen, insbesondere 1 bis 4 C-Atomen, vorzugsweise 1 bis 3 C-Atomen, substituiert sein. Beispiele für Alkyl- und Alkoxyalkylreste, die für $R^4$ stehen können, sind : Methyl ; Ethyl ; n-Propyl ; i-Propyl ; n-, i-, sec.- und tert.-Butyl ; n- und i-Pentyl ; n- und i-Hexyl ; Methoxy-, Ethoxy-, n-Propoxy-, i-Propoxy-, n-Butoxy-, i-Butoxymethyl ; 2-Methoxy-, 2-Ethoxy-, 2-n-Propoxy-, 2-i-Propoxy-, 2-n-Butoxy-ethyl ; 2-Methoxy-, 3-Ethoxy-, 3-n-Propoxy-, 3-i-Proppoxy-propyl oder -i-propyl. Die für $R^4$ stehenden aliphatischen Reste, insbesondere die Alkylreste, können auch mit einem aliphatisch substituierten Thiorest mit bis zu 4 C-Atomen substituiert sein. Derartige aliphatische Thioreste sind zum Beispiel Alkylthioreste mit 1 bis 4 C-Atomen, wie zum Beispiel Methyl-, Ethyl-, n-Propyl-, i-Propyl-, n-Butyl-, tert.-Butyl-thio, vorzugsweise aber Allylthio ($CH_2 = CH-CH_2-S-$). Als für $R^4$ stehende cycloaliphatische Reste kommen vor allem Cycloalkylreste mit 5 bis 7 C-Atomen, insbesondere Cyclopentyl, und bevorzugt Cyclohexyl, in Betracht. Als für $R^4$ stehender bicycloaliphatischer Rest kommt insbesondere das 2,6,6-Trimethylbicyclo(3.1.1)heptan-3-yl (= Pinanyl-3) in Betracht. Als für $R^4$ stehender tricycloaliphatischer Rest kommt insbesondere das Tricyclo($3.3.1.1^{3,7}$)decan-1-yl (= Adamant-1-yl) in Betracht.

Die Alkoxysubstituenten für die Alkoxyreste besitzen insbesondere 1 bis 4 C-Atome. Beispiele für Alkoxyreste und Alkoxyalkoxyreste, die für $R^4$ stehen können sind : Methoxy ; Ethoxy ; n- und i-Propoxy ; n-, i-, sec.- und tert.-Butoxy ; n-Pentyloxy ; i-Hexyloxy ; n-Octyloxy ; n-Dodecyloxy ; n-Hexadecyloxy ; n-Heptadecyloxy ; n-Octadecyloxy ; Methoxy-, Ethoxy-, n-Propoxy-, i-Propoxy-, n-Butoxy-methoxy ; 2-Methoxy-, 2-Ethoxy-, 2-N-Propoxy-, 2-i-Propoxy-ethoxy ; 3-Methoxy-, 3-Ethoxy-, 3-n-Propoxy-, 3-i-Propoxy-propoxy ; 4-Methoxy-, 4-Ethoxy-, 4-n-Propoxy-, 3-Propoxy-, 4-n-Butoxy-butoxy.

Der für $R^4$ stehende Alkoxycarbonylrest besitzt vorzugsweise 2 bis 5 C-Atome. Beispielsweise seien hierfür genannt : Methoxy-, Ethoxy-, n-Propoxy-, i-Propoxy-, n-Butoxy-, i-Butoxy-carbonyl. Als für $R^4$ stehender Alkoxycarbonylrest kommt insbesondere der Ethoxycarbonylrest in Betracht.

Als für $R^4$ stehende substituierte und unsubstituierte Arylreste sind z.B. α- oder β-Naphthylreste, insbesondere aber der Phenylrest, zu nennen. Als für $R^4$ stehende Aryloxyreste sind z.B. α-oder β-Naphthoxyreste, insbesondere aber der Phenoxyrest, zu nennen. Die für $R^4$ stehenden Arylreste können mono-, di- oder trisubstituiert sein, wobei jedoch auch bei einer Trisubstitution nur maximal 2 Nitrogruppen vorhanden sein können, wie beispielsweise 2-Methyl-4,6-dinitrophenyl und 2-Chlor-6-methyl-4-nitrophenyl. Bei sperrigen Substituenten ist evtl. nur eine Di- oder Monosubstitution möglich. Als Halogensubstituenten für die Arylreste kommen z.B. Fluor-, Chlor- und/oder Bromatome in Betracht. Als Alkylcarbonyloxysubstituenten für die Arylreste, insbesondere für einen Phenylrest, sind z.B. zu nennen : Acetoxy, n-Propionyloxy, i-Propionyloxy, n-Butyryloxy, i-Butyryloxy.

Beispiele für die für $R^4$ stehenden, gegebenenfalls substituierten Arylreste sind : Phenyl, 2-, 3- oder 4-Methyl-, -Ethyl-, -n-Propyl-, -i-Propyl-phenyl ; 2-, 3- oder 4-Methoxy-, -Ethoxy-, -n-Propoxy-, -i-Propoxy-phenyl ; 2-, 3- oder 4-Fluor-, -Chlor- oder -Brom-phenyl ; 2-, 3- oder 4-Nitrophenyl ; 2-, 3- oder 4-Hydroxyphenyl ; 2-, 3- oder 4-Acetoxy-, -n-Propionyloxy-, -n-Butyryloxy-phenyl ; 2,3-, 2,4-, 2,5- oder 2,6-Dimethyl-, -Diethyl-, -Dipropyl-phenyl ; 2- oder 3-Methyl-4-chlorphenyl ; 2- oder 3-Ethyl-4-fluorphenyl ; 2-Chlor-4-ethylphenyl ; 2-Brom-4-i-propylphenyl ; 2,6-Diethoxy-4-chlorphenyl ; 2,3,4-, 3,4,5- oder 2,3,5-Trimethyl-, -Triethyl-, -Tripropyl-, -Trimethoxy-, -Triethoxy- oder Tripropoxy-phenyl ; 2-Hydroxy-3-, -4- oder -5-chlorphenyl ; 2-Methyl-3-, -4- oder -5-acetoxy-phenyl.

Als für $R^4$ stehende substituierte Arylreste sind insbesondere zu nennen : Methylphenyl (= Tolyl), Methoxyphenyl und Chlorphenyl. Der für $R^4$ stehende Imidazolylrest ist vorzugsweise ein 1-Imidazolylrest.

Für $R^4$ sind bevorzugt : Methyl, Ethyl, Cyclohexyl, Phenyl, 4-Chlorphenyl, 4-Methoxyphenyl, Methoxy, Ethoxy, n-Propoxy, i-Propoxy, n-Decyloxy, n-Octadecyloxy, 2-n-Propoxy-ethoxy, 2-i-Propoxy-ethoxy, n-Butoxymethyl, 2-n-Butoxy-ethoxy und Allylthiomethyl.

n ist vorzugsweise die Zahl 2.

Bevorzugte Verbindungen der Formel I sind solche, bei denen A eine direkte Bindung, $-CH_2-$ oder $-S(O_2)-$, $R^2$ Methyl und $R^1$ Wasserstoff, -NO, $-COR^4$ mit $R^4$ = Methyl, Ethyl, Methoxy, Ethoxy, n- und i-Propoxy, Phenyl, Cyclohexyl, Allylthiomethyl, n-Butoxymethyl, 2-n-Butoxy-ethoxy bedeuten.

Eine Verbindung der allgemeinen Formel I kann dadurch hergestellt werden, daß eine Verbindung der allgemeinen Formel II

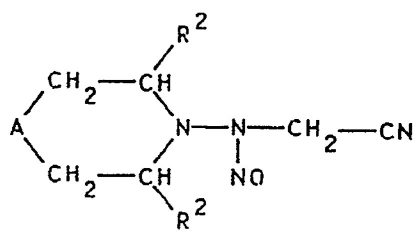

(II)

worin A und $R^2$ die bereits genannten Bedeutungen besitzen, zu einer Verbindung der allgemeinen Formel Ia

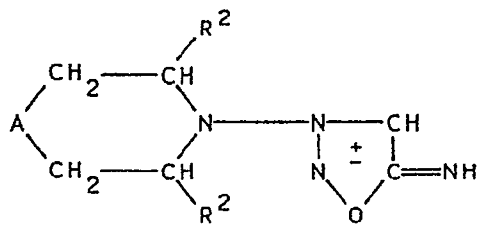

(Ia)

cyclisiert wird und daß diese oder eine Säureadditionssalz davon für den Fall, daß eine Verbindung der Formel I mit $R^1 = -COR^4$ hergestellt werden soll, mit einem Acylierungsmittel, das den Rest $-COR^4$ einführt, acyliert wird oder für den Fall, daß eine Verbindung der Formel I mit $R^1 = -NO$ hergestellt werden soll, nitrosiert wird und die so erhaltene Verbindung gegebenenfalls in ein pharmakologisch annehmbares Säureadditionssalz überführt wird.

Die Cyclisierung der Verbindung II zu der Verbindung Ia wird in einem geeigneten organischen oder anorganischen Lösungs-, Dispergier- oder Verdünnungsmittel unter Zusatz eines Cyclisierungsmittels, normalerweise bei Temperaturen von −10 bis 40°C, insbesondere 0 bis 40°C, vorzugsweise bei 0 bis 20°C, durchgeführt.

Als Cyclisierungsmittel sind solche geeignet, die in wäßriger Lösung einen pH-Wert von 3 oder darunter einstellen, also z.B. starke Säuren, wie Mineralsäuren, wie Schwefel-, Salpeter- oder Phosphorsäure, vorzugsweise Chlorwasserstoff, aber auch starke organische Säuren, wie Sulfonsäuren oder Trifluoressigsäure. Die Cyclisierung wird normalerweise unter Eiskühlung durchgeführt. Von dem Cyclisierungsmittel kommt z.B. bezogen auf 1 mol der Verbindung der Formel II 0,1 bis 10 mol, vorzugsweise 1 bis 5 mol, zur Anwendung. Das Cyclisierungsmittel wird normalerweise im Überschuß eingesetzt. Besonders bequem ist die Verwendung von Chlorwasserstoff als Cyclisierungsmittel, der normalerweise bis zur Sättigung in den Reaktionsansatz eingeleitet wird. Bei der Cyclisierung wird normalerweise das entsprechende Säureadditionssalz der Verbindung Ia erhalten.

Geeignete Lösungs-, Dispergier- oder Verdünnungsmittel sind z.B. : Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol ; Ether, insbesondere solche mit 2 bis 8 C-Atomen im Molekül, wie z.B. Diethylether, Methylethylether, Di-n-propyl-ether, Di-isopropyl-ether, Methyl-n-butylether, Methyl-tert-butylether, Ethyl-propylether, Di-butyl-ether, Tetrahydrofuran, 1,4-Dioxan, 1,2-Dimethoxyethan, Bis-β-methoxyethylether ; Oligoethylen-glykol-dimethyl-ether, wie z.B. Tetraglyme oder Pentaglyme ; Carbonsäurealkylester, insbesondere solche mit 2 bis 10 C-Atomen im Molekül, wie z.B. Ameisensäure-methyl-, -ethyl-, -butyl- oder -isobutylester, Essigsäure-methyl-, -ethyl-, -propyl-, isopropyl-, -butyl-, -isobutyl- oder -sec-butyl-, -amyl-, -isoamyl-, -hexyl-, -cyclohexyl- oder -benzyl-ester, Propionsäure-methyl-, -ethyl- oder -butyl-ester ; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Dimethylcyclohexanon, Benzophenon, Acetophenon ; aliphatische Kohlenwasserstoffe, wie z.B. Hexan, Heptan, niedrig-und hochsiedende Petrolether, Spezialbenzine und Testbenzin ; cycloaliphatische Kohlenwasserstoffe, wie z.B. Cyclopentan, Cyclohexan, Methylcyclohexan, Tetralin, Decalin ; aromatische Kohlenwasserstoffe, wie z.B. Benzol, Toluol, -o, m- und p-Xylol, Ethylbenzol ; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol ; Hexamethylphosphorsäuretriamid ; Sulfoxide, wie z.B. Dimethyl-sulfoxid ; Tetramethylensulfon ; Wasser. Auch Gemische verschiedener Lösungs- oder Dispergiermittel können verwendet werden, beispielsweise Wasser-Methanol oder vorzugsweise Essigsäureethylester-Methanol.

Die Verbindungen der Formel Ia stellen erfindungsgemäße Verbindungen der allgemeinen Formel I für den Fall dar, daß $R^1$ = Wasserstoff ist.

Die Acylierung der Verbindung der Formel Ia, die auch in Form eines Säureadditionssalzes vorliegen kann, zur Einführung des Restes $R^1 = -COR^4$ kann in an sich bekannter Weise mit einem geeigneten Acylierungsmittel der Formel III durchgeführt werden

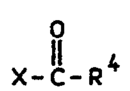

(III),

worin X ein nukleophil abspaltbarer Rest darstellt.

In der Formel III bedeutet X z.B. insbesondere Halogen, vorzugsweise –Cl oder –Br ; –OH ; –O-Alkyl, insbesondere mit 1 bis 5 C-Atomen ; –O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyl- oder Nitrophenyl-Rest ist ; $-O-CO-R^4$ ; –O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasi-aromatischen Fünfring.

Die Acylierung wird zweckmäßigerweise in flüssiger Phase in Anwesenheit eines inerten Lösungs-, Dispergier- oder Verdünnungsmittels oder in einem Überschuß des Acylierungsmittels, zweckmäßigerweise unter Rühren, durchgeführt.

Bei der Acylierung beträgt das Molverhältnis zwischen der Verbindung der Formel Ia und dem Acylierungsmittel der Formel III theoretisch 1 : 1. Zweckmäßigerweise wird das Acylierungsmittel der Formel III in einem geringen molaren Überschuß eingesetzt. Überschüsse von bis zu 30 mol% sind in der Regel ausreichend, d.h. das Molverhältnis zwischen der Verbindung der Formel Ia und dem Acylierungsmittel der Formel III beträgt normalerweise 1 : (1 bis 1,3), vorzugsweise 1 : (1 bis 1,2). Falls bei der Acylierungsreaktion eine Säure abgespalten wird, ist der Zusatz eines Säurefängers, wie z.B. eines Alkylihydroxids, wie z.B. Natrium-, Kalium- oder Lithiumhydroxid, eines tertiären organischen Amins, wie z.B. Pyridin oder Triethylamin, eines Alkalicarbonats oder Alkalibicarbonats, wie z.b. Soda oder Natriumbicarbonat, oder eines Alkalisalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Acylierung kann prinzipiell bei Temperaturen zwischen –10°C und em Siedepunkt des verwendeten Lösungs-, Dispergier- oder Verdünnungsmittels erfolgen. In vielen Fällen wird die Umsetzung bei 0 bis 50°C, insbesondere bei 0 bis 30°C und vorzugsweise bei Raumtemperatur, durchgeführt.

Die Verbindungen der Formel III sind Acylierungsmittel und stellen somit z.B. dar : für X = Halogen Säurehalogenide bzw. Halogenameisensäureester, von denen Säurechloride und Chlorameisensäureester bevorzugt sind ; für –OH Carbonsäuren ; für –O-Alkyl und –O-Aryl Ester, von denen die Tolyl-, 2,4-Dinitro- oder 4-Nitrophenylester – bevorzugt sind ; für $-O-CO-R^4$ Anhydride ; für –O-CO-O-Alkyl gemischte Carbonsäure-Kohlensäure-Anhydride ; oder heterocyclische Amide oder Azolide, insbesondere von N,N'-Carbonyldiazolen, wie z.B. N,N'-Carbonyldiimidazol, 2,2'-Carbonyl-ditriazol(1,2,3), 1,1'-Carbonyl-ditriazol(1,2,4), N,N'-Carbonyldipyrazol, 2,2'-Carbonyl-ditriazol (vgl. z.B. H.A. Staab, M. Lücking und F.H. Dürr, Chem. Ber. 95, (1962), 1275 ff, H. A. Staab und A. Mannschreck, Chem. Ber. 95, (1962), 1284 ff. ; H.A. Staab und W. Rohr, "Synthesen mit heterocyclischen Amiden (Azoliden)" in "Neuere Methoden der Präparativen Organischen Chemie", Band V, Verlag Chemie, 1967, S. 53 ff., insbesondere S. 65 bis 69). Die Acylierungsmittel der Formel III können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel III zu überführen. Als derartige Aktivierungsmittel sind z.B. geeignet : N,N'-disubstituierte Carbodiimide, insbesondere wenn sie mindestens einen sekundären oder tertiären Alkylrest enthalten, wie z.B. Diisopropyl-, Dicyclohexyl- oder N-Methyl-N'-tert.butylcarbodiimid (vgl. Methodicum Chimicum, Verlag G.Thieme, Stuttgart, Bd.6, (1974), S.682/683, und Houben-Weyl, Methoden der Org. Chemie, Bd.8, (1952), S. 521/522) ; Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368) ; Kohlensäureester, wie z.B. N,N'-Disuccinimido-carbonat, Diphthalimido-carbonat, 1,1'-(Carbonyldioxy)-dibenzo-triazol oder Di-2-pyridyl-carbonat (vgl. z.B. Tetrahedron Letters, Vol.25, No. 43, 4943-4946), gegebenenfalls in Anwesenheit geeigneter Katalysatoren, wie z.B. 4-Dimethylaminopyridin. Ferner sind als Aktivierungsmittel N,N'-Carbonyldiazole, wie z.B. N,N'-Carbonyl-diimidazol, 2,2'-Carbonyl-ditriazol(1,2,3), 1,1'-Carbonyl-ditriazol(1,2,4), N,N'-Carbonyl-dipyrazol, 2,2'-Carbonyl-ditetrazol, N,N'-Carbonyl-benzimidazol oder N,N'-Carbonylbenztriazol, geeignet (vgl. z.B. H.A. Staab, M. Lücking und F.H. Dürr, loc. cit ; H.A. Staab und A. Mannschreck loc. cit. ; H.A. Staab und W. Rohr loc. cit). Als N,N'-Carbonyl-diazol wird häufig das käufliche N,N'-Carbonyl-diimidazol verwendet. Die anderen N,N'-Carbonylazole sind aber aus dem jeweiligen Azol und Phosgen ebenfalls leicht zugänglich.

Als Aktivierungsmittel für Carbonsäuren sind ferner geeignet : Derivate der Oxalsäure, wie z.B. Oxalylchlorid (vgl. z.B. GP-PS 2 139 225) oder N,N'-Oxalyl-diazole wie z.B. 1,1'-Oxalyldi-imidazol, 1,1'-Oxalyldi-1,2,4-triazol und 1,1'-Oxalyldi-1,2,3,4-tetrazol (vgl. z.B. Shizuaka Murata, Bull.Chem. Soc.Jap. 57, 3597-3598 (1984)) ; Methylethylphosphinsäureanhydrid (vgl. z.B. DE-OS 31 01 427) ; Diphosphortetrajodid (Chem. Lett. 1983, 449) ; Dialkyldisulfit (Indian J. Chem. 21, 259 (1982)) ; oder andere reaktive Agentien.

Geeignete Lösungs-, Dispergier- oder Verdünnungsmittel für die Acylierung sind z.B. solche, die für die

Durchführung der Cyclisierung angegeben worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Falls eine Verbindung der Formel I mit $R^1 = -NO$ hergestellt werden soll, wird eine Verbindung der Formel Ia, die auch in Form eines Säureadditionssalzes vorliegen kann, in an sich bekannter Weise, zweckmäßigerweise in einem geeigneten inerten Lösungsmittel oder Lösungsmittelgemisch, vorzugsweise in Wasser, normalerweise bei Temperaturen von 0 bis 40°C, und vorzugsweise bei Temperaturen von 0 bis 10°C, nitrosiert. Die Nitrosierung erfolgt z.B. mit salpetriger Säure, NO, NOCl oder NO-haltigen Gasgemischen. Zweckmäßigerweise erfolgt die Nitrosierung mit salpetriger Säure, die vorteilhafterweise aus einem Alkalimetallnitrit, z.B. Natriumnitrit, und eine Säure, insbesondere Salzsäure, erzeugt wird. Es ist zweckmäßig, die wäßrige Lösung der Verbindung Ia mit einer Säure, insbesondere Salzsäure, auf einen pH-Wert von 1 bis 3 einzustellen und das Alkalimetallnitrit in Form einer wäßrigen Lösung zu der gerührten und abgekühlten Lösung der Verbindung zuzutropfen.

Die erfindungsgemäßen Verbindungen der Formel I können in verschiedenen Konfigurationen in cis- oder in trans-Form vorliegen, wobei die trans-Formen als racemische Gemische oder in optisch aktiver Form vorliegen können. Für die Herstellung dieser verschiedenen Isomeren können an sich bekannte Verfahren, wie selektive Synthese, chirale Synthese oder Trennung von racemischen Gemischen nach bekannten Methoden Anwendung finden.

Die substituierten 3-Amino-sydnonimine der allgemeinen Formel I bilden mit anorganischen oder organischen Säuren Säureadditionssalze. Zur Bildung derartiger Säureadditionssalze sind anorganische oder organische Säuren geeignet. Geeignete Säuren sind beispielsweise Chlorwasserstoff, Bromwasserstoff, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen-, Adipinsäure oder Naphthalindisulfonsäuren, insbesondere Naphthalindisulfonsäure(1,5). Pharmakologisch annehmbare Säureadditionssalze werden bevorzugt. Die Säureadditionssalze können wie üblich durch Vereinigung der Komponenten, zweckmäßigerweise in einem geeigneten Lösungs- oder Verdünnungsmittel, hergestellt werden. Bei der Synthese der Verbindungen der Formel Ia fallen die Säureadditionssalze an.

Die benötigten Ausgangsverbindungen der allgemeinen Formel II können in an sich bekannter Weise nach der Strecker'schen Aminonitrilsynthese aus Verbindungen der allgemeinen Formel IV

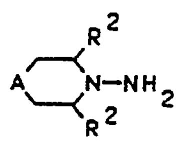

(IV)

worin A und $R^2$ die bereits genannten Bedeutungen besitzen, durch Umsetzung mit Formaldehyd und Blausäure bzw. Natriumcyanid in einem geeigneten Lösungsmittel, z.B. Wasser, hergestellt werden, wobei zunächst eine Verbindung der allgemeinen Formel V

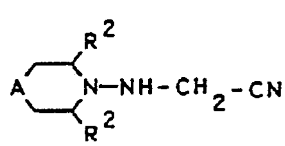

(V)

entsteht, die durch Nitrosierung in die Verbindung II überführt wird. Die Nitrosierung wird in bekannter Weise, zweckmäßigerweise in einem geeigneten inerten Lösungsmittel oder Lösungsmittelgemisch, vorzugsweise in Wasser, normalerweise bei Temperaturen von 0 bis 40°C und vorzugsweise bei Temperaturen von 0 bis 10°C, durchgeführt. Die Nitrosierung erfolgt z.B. mit salpetriger Säure, NO, NOCl oder NO-haltigen Gasgemischen. Zweckmäßigerweise erfolgt die Nitrosierung mit salpetriger Säure, die vorteilhafterweise aus einem Alkalimetallnitrit, z.B. Natriumnitrit, und einer Säure, insbesondere Salzsäure, erzeugt wird. Es ist zweckmäßig, die wäßrige Lösung der Verbindung V mit einer Säure, insbesondere Salzsäure, auf einen pH-Wert von 1 bis 3 einzustellen und das Alkalimetallnitrit in Form einer wäßrigen Lösung zu der gerührten und abgekühlten Lösung der Verbindung zuzutropfen.

Aus der Lösung der dabei erhaltenen Verbindung II kann die Verbindung II isoliert werden, oder die Lösung der Verbindung II kann direkt der Cyclisierungsreaktion unterworfen werden. Normalerweise ist es jedoch für die anschließende Cyclisierung angebracht, die Nitrosoverbindung II zunächst in einem geeigneten organischen Lösungsmittel aufzunehmen und in ihm, gegebenenfalls nach Zusatz eines weiteren Lösungsmittels, die Cyclisierung zu der Verbindung der Formel Ia durchzuführen.

Die Verbindungen der allgemeinen Formel IV sind zum Teil bekannt (vergl. z.B. C.G. Overberger et al, J. Amer. Chem. Soc. 77, (1955) 4100) bzw. lassen sich, ausgehend von Verbindungen der allgemeinen Formel VI

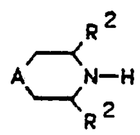

(VI)

dadurch herstellen, daß entweder

a) eine Verbindung der Formel VI zur N-Nitrosoverbindung VII nitrosiert und anschließend, zweckmäßigerweise mit Lithiumaluminiumhydrid, reduziert wird :

(VI)
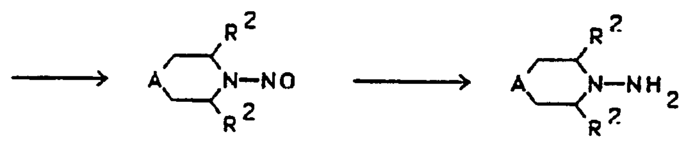

(VII) (IV)

oder daß in an sich bekannter Weise

b) eine Verbindung der Formel VI mit Kaliumcyanat im sauren Medium in das Harnstoffderivat VIII überführt wird, das dann durch Oxydation mit Natriumhypochlorit nach dem Hoffmann-Abbau in die Verbindung IV überführt wird :

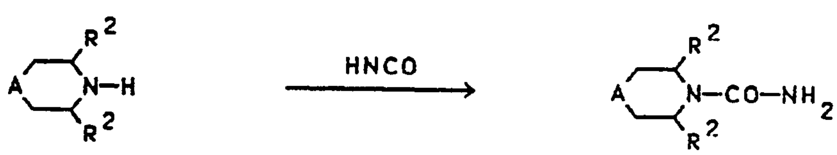


(VI) (VIII)

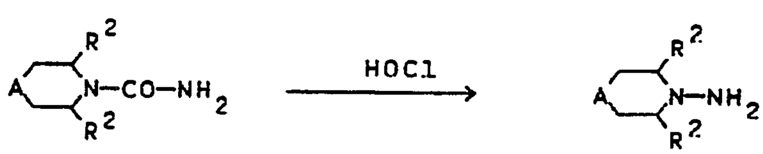


(VIII) (IV)

Ausgangsverbindungen der Formel VI sind bekannt (vergl. z.B. Backer, van der Ley, Rec. Trav. Chim. Pays-Bas 70, (1951) 564 ; Berlin, Sytschewa, Z. obsc. Chim. 20, (1950) 640 ; Idson, Spoerri, J. Amer. Chem. Soc. 76, (1954) 2902) bzw. lassen sich nach den für diese Verbindungsklassen bekannten Verfahren herstellen. So lassen sich Verbindungen der Formel VI z.B. aus Verbindungen der allgemeinen Formel IX

$R^{2*} = CH_{-2}\text{-}A\text{-}CH_2\text{-}CH = R^{2*}$ (IX)

worin A –SO– oder –$S(O_2)$– und

$R^{2*}$ einen Rest bedeutet, der bei der Umsetzung mit Ammoniak durch Addition von Wasserstoff in den bereits genannten Rest $R^2$ übergeht und die nach an sich bekannten Methoden darstellbar sind, durch Ringschluß mit Ammoniak herstellen. Die Umsetzung mit Ammoniak kann bei Temperaturen von 20 vis 150°C, vorzugsweise bei 60 bis 100°C, mit oder ohne Lösungsmittel durchgeführt werden.

Führt man die Umsetzung der Verbindungen der Formel IX nicht mit Ammoniak sondern unter den vorgenannten Bedingungen mit Hydrazin durch, so kann man bei dem Ringschluß direkt zu Verbindungen der Formel IV gelangen.

Die Verbindung der allgemeinen Formel I und ihre pharmakologisch annehmbaren Säureadditionssalze besitzen wertvolle pharmakologische Eigenschaften. Besonders ausgeprägt ist ihre Wirkung auf das Herz-Kreislauf-System. Verglichen mit bekannten, in 3- Stellung substituierten Sydnoniminverbindungen, z.B. solchen der EP-B-59356, sowie der im Handel befindlichen strukturähnlichen Verbindung Molsidomin, besitzen sie z.B. überraschenderweise eine wesentlich längere Wirkungsdauer. Sie senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise : β-Rezeptorenblocker, wie z.B. Propranolol, Pindolol, Metoprolol ; Vasodilatatoren, wie z.B. Carbochromen ; Beruhigungsmittel, wie z.B. Barbitursäurederivate, 1,4-Benzodiazepine und Meprobamat ; Diuretica, wie z.B. Chlorothiazid ; das Herz tonisierende Mittel, wie z.B. Digitalispräparate ; blutdrucksenkende Mittel, wie z.B. Hydralazin, Dihydralazin, Prazosin, Clonidin, Rauwolfia-Alkaloide ; Mittel, die den Fettsäurespiegel im Blut senken, wie z.B. Bezafibrat, Fenofibrat; Mittel für die Thromboseprophylaxe, wie z.B. Phenprocoumon, außerdem N-substituierte-N-Nitroso-aminoacetonitrile der Formel II, wobei A und $R^2$ die genannten Bedeutungen besitzen und wobei A insbesondere $-CH_2-$, $-S(O_2)-$, $-O-$ oder eine direkte Bindung und/oder $R^2$ Methyl bedeutet. Die N-substituierten-N-Nitroso-aminoacetonitrile der Formel II besitzen ähnlich günstige pharmakologische Eigenschaften wie die Verbindungen der Formel I.

Die Verbindungen der Formel I, ihre pharmakologisch annehmbaren Säureadditionssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Zum Nachweis der antianginösen Wirkung der erfindungsgemäßen Verbindungen wurden Untersuchungen an Bastardhunden beiderlei Geschlechts in Pentobarbital-Narkose (30 bis 40 mg/kg i.v.) oder in Urethan-Chloralose-Narkose (3 ml/kg Urethan-Chloralose-Gemisch i.v. = 20 mg/kg Chloralose und 250 mg/kg Urethan) durchgeführt. Die Beatmung der Tiere erfolgte mit einem Bird-Mark-7-Respirator. Der endexpiratorische Kohlensäuregehalt (gemessen mit einem Ultrarotabsorptionsschreiber) betrug zwischen 4,5 und 5 Vol.%. Während des gesamten Versuchs erhielten die Tiere mit Pentobarbital-Narkose eine Dauerinfusion von Pentobarbital i.v. = 4 mg (in 6 ml)/kg/h, um eine konstante Narkosetiefe zu gewährleisten. Die Tiere mit Urethan-Chloralose-Narkose erhielten keine Dauerinfusion. Die Infusion wurde durch die Vena cephalica gegeben. Nach der Präparation des Versuchstieres wurde ca. 1 Stunde gewartet, bis sich alle haemodynamischen Parameter eingestellt hatten (steady state). Danach wurde mit dem eigentlichen Versuch begonnen.

Zur Bestimmung des mittleren peripheren Blutdrucks (= BD) wurden der systolische und diastolische Blutdruck peripher in der Arteria femoralis über einen Statham-Druckaufnehmer gemessen. Ein über die Arteria carotis in den linken Ventrikel geschobener Millar-Tip-Katheter lieferte das Signal für den linksventrikulären enddiastolischen Druck (= LVEDP) und die Herzfrequenz (= HF).

Die erhaltenen Ergebnisse sind in der nachfolgenden Tabelle angegeben.

| Substanz | Dosis mg/kg | BD Δmm Hg | LVEDP Δmm Hg | HF Δ b/min | WD min |
|---|---|---|---|---|---|
| A | 0,3 | -30 | -3 | +5 | 180 |
| B | 0,3 | -25 | -3 | +5 | 180 |
| C | 0,3 | -35 | -4 | +7 | 200 |
| D | 0,3 | -25 | -4,5 | +4 | 180 |
| E | 0,3 | -35 | -4 | +5 | 150 |
| F | 0,3 | -40 | -7 | +8 | 150 |
| Mol | 0,1 | -18 | -2,3 | +3 | 90 |

In der vorstehenden Tabelle bedeuten :

A = 3-(2,6-Dimethylpiperidin-1-yl)-sydnonimin-hydrochlorid
B = 3-(2,6-Dimethylpiperidin-1-yl)-N-benzoyl-sydnonimin
C = 3-(2,6-Dimethylpiperidin-1-yl)-N-(allylthio)-acetyl-sydnonimin
D = 3-(2,6-Dimethylpiperidin-1-yl)-N-i-propoxycarbonyl-sydnonimin
E = 3-(2,6-Dimethylpiperidin-1-yl)-N-butoxy-acetyl-sydnonimin
F = 3-(2,5-Dimethylpyrrolidin-1-yl)-sydnonimin-hydrochlorid
Mol = Vergleichssubstanz Molsidomin (3-(Morpholin-1-yl)-N-ethoxycarbonyl-sydnonimin)
BD = Mittlerer peripherer Blutdruck
LVDEP = Linksventrikulärer enddiastolischer Druck
HF = Herzfrequenz (b/min = Schläge pro Minute)
WD = Wirkungsdauer

Die Vergleichssubstanz Molsidomin ist in Beispiel 4 der USP-3769283 beschrieben.

Die DE-A 1620501 betrifft Sydnoniminderivate, die in 3-Stellung einen $R^1$ ($R^2$) N-Rest besitzen, wobei $R^1$ und $R^2$ zusammen unter anderem den Morpholinorest, den Pipecolinorest, einen Piperidinorest oder einen in 4-Stellung durch Alkyl substituiertn Piperazinorest bedeuten können. Durch die Entgegenhaltung werden die erfindungsgemaßen Verbindungen nicht nahegelegt, weil der Entgegenhaltung keinerlei Hinweise auf 2, 6-dialkylsubstituierte heterocyclische Reste in 3-Stellung des Sydnonimins zu entnehmen sind.

Die erfindungsgemäßen Verbindungen werden auch nicht durch eine Kombination der entgegengehaltenen DE-A-1620501 und der entgegengehaltenen FR-A-2271818 nahegelegt. Die FR-A-2271818 betrifft in 2-Stellung substituierte 5,6-Dimethoxyindazole, die nach den Angaben in Anspruch 1 in 2-Stellung durch 15

verschiedene Reste substituiert sein können Der Anspruch 15 der FR-A-2271818 betrifft das 2-(2,6-Dimethylpiperidino)-5,6-dimethoxyindazol. In Anbetracht der Vielzahl der in 2-Stellung der Indazole der FR-A-2271818 möglichen Substituenten und des anderen Grundgerüstes zwischen Indazolen und Sydnoniminen konnte der Fachmann aus den Entgegenhaltungen keine Hinweise darauf entnehmen, bei der Entwicklung neuer, pharmakologisch wertvoller Sydnoniminverbindungen einen 2,6-Dimethylpiperidinorest in 3-Stellung vorzusehen.

Von den erfindungsgemäßen Verbindungen der Formel I sind 3-(2,6-Dimethylpiperidin-1-yl)-sydnonimin und seine pharmakologisch annehmbaren Säureadditionssalze, insbesondere das Hydrochlorid, sowie 3-(2,6-Dimethylpiperidin-1-yl)-N-benzoyl-sydnonimin und 3-(2,6-Dimethylpiperidin-1-yl)-N-allylthio-methylcarbonyl-sydnonimin (= 3-(2,6-Dimethylpiperidin-1-yl)-N-(allylthio)-acetyl-sydnonimin) und deren Säureadditionssalze bevorzugt.

In den nachfolgenden Beispielen sind, sofern nichts Anderes angegeben ist, Prozente in Gewichtsprozenten angegeben. Angegebene Verhältnisse zwischen Komponenten von Lösungs- oder Fließmitteln sind Volumenverhältnisse. Die Angabe Fp. bedeutet Schmelzpunkt und "Zers." bedeutet Zersetzung.

Beispiel 1

3-(2,6-Dimethylpiperidin-1-yl)-sydnonimin-hydrochlorid

a) Zur Mischung von 29,1 g N-(2,6-Dimethylpiperidin-1-yl)-aminoacetonitril, 100 ml Wasser, 100 ml Essigsäureethylester und 12 g konz. Salzsäure wird die Lösung von 13,4 g Natriumnitrit in 10 ml Wasser getropft und nach 5 Stunden Rühren bei Raumtemperatur die organische Phase abgetrennt, mit 20 ml Methanol verdünnt und im Eisbad gerührt. Nach dem Einleiten von Salzsäure bis zur Sättigung wird 15 Stunden bei Raumtemperatur nachgerührt und im Wasserstahl-Vakuum eingeengt. Der Rückstand kristallisiert beim Verrühren mit Diethylether. Er wird abgesaugt und aus Acetonitril umkristallisiert.
Ausbeute : 18,2 g 3-(2,6-Dimethylpiperidin-1-yl)-sydnonimin-hydrochlorid
Schmelzpunkt : 136-137°C (Zers.)
b) Das vorstehend bei a) benötigte Ausgangsprodukt 3-(2,6-Dimethylpiperidin-1-yl)-aminoacetonitril wird wie folgt hergestellt :
Zur eisgekühlten Mischung von 30,0 g 1-Amino-2,6-dimethylpiperidin, 20 g konz. Salzsäure und 100 ml Wasser wird die Lösung von 11,5 g Natriumcyanid in 20 ml Wasser gegeben und der pH-Wert mit Salzsäure auf 6,5 eingestellt. Dann werden 19,3 g einer 39%igen wäßrigen Formaldehyd-Lösung zugesetzt und die Reaktionsmischung 3 Stunden bei 0°C und weitere 3 Stunden bei Raumtemperatur gerührt. Das Produkt wird mit Essigsäure-ethylester extrahiert, die Essigsäure-ethylesterphase mit verdünntem Eisessig gewaschen und über Natriumsulfat getrocknet. Nach dem Einengen verbleibt ein öliger Rückstand (29,1 g), der ohne weitere Reinigung gemäß a) weiterverarbeitet wird.

Beispiel 2

3-(2,6-Dimethylpiperidin-1-yl)-N-ethoxycarbonylsydnonimin

Zur eisgekühlten Lösung von 5 g 3-(2,6-Dimethylpiperidin-1-yl)-sydnonimin-hydrochlorid, hergestellt nach Beispiel 1a) in 20 ml Wasser werden 3,5 g Natriumbicarbonat gegeben und anschließend die Lösung von 2,5 g Chlorameisensäure-ethylester in 20 ml Methylenchlorid zugefügt und die Mischung 4 Stunden bei Raumtemperatur gerührt. Die organische Phase wird abgetrennt, getrocknet und eingeengt. Der Rückstand wird mit Petrolether verrieben und abgesaugt.
Ausbeute : 4,2 g
Schmelzpunkt : 90 bis 91°C

Beispiel 3

3-(3,5-Dimethyl-thiomorpholin-1,1-dioxid-4-yl)-sydnonimin-hydrochlorid

a) Die Mischung aus 32,2 g Diallylsulfon, 55 g Hydrazinhydrat und 54 ml Wasser wird 30 Minuten zum Sieden erhitzt. Der Ansatz wird im Eisbad gerührt, der Niederschlag abgesaugt und aus Ethanol umkristallisiert.
Ausbeute : 16,2 g 4-Amino-3,5-dimethyl-thiomorpholindioxid
Schmelzpunkt : 181 bis 183°C
b) Die Mischung aus 16,2 g 4-Amino-3,5-dimethyl-thiomorpholin-dioxid, 9,1 g konz. Salzsäure, 80 ml Was-

ser und 5,6 g Natriumcyanid wird auf 5°C abgekühlt und mit 9,1 g einer 39%igen Formaldehydlösung versetzt (pH = 7 bis 7,5). Die Reaktionsmischung wird dann 4 Stunden bei Raumtemperatur gerührt. Unter Eiskühlung wird die Mischung sauer (pH = 1) gestellt und eine Lösung von 6,3 g Natriumnitrit in 25 ml Wasser zugetropft. Die Mischung wird bei Raumtemperatur noch 3 Stunden nachgerührt und der Feststoff abgesaugt.
Ausbeute : 16,0 g N-(3,5-Dimethyl-thiomorpholin-1,1-dioxid-4-yl)-N-nitroso-aminoacetonitril
Schmelzpunkt : 163 bis 165°C

c) 15,8 g N-(3,5-Dimethyl-thiomorpholin-1,1-dioxid-4-yl)-N-nitroso-aminoacetonitril werden in 100 ml Methanol und 100 ml Essigsäure-ethylester gelöst und mit HCl gesättigt. Vom ausgefallenen Feststoff wird abgesaugt und das Filtrat eingeengt. Der Rückstand wird mit absolutem Ethanol und mit Essigsäure-ethylester verrührt, abgesaugt und getrocknet.
Ausbeute : 8,5 g 3-(3,5-Dimethyl-thiomorpholin-1,1-dioxid-4-yl)-sydnonimin-hydrochlorid
Schmelzpunkt : 192°C (Zers.)

Beispiel 4

N-Ethoxycarbonyl-3-(3,5-dimethyl-thiomorpholin-1,1-dioxid-4-yl)-sydnonimin.

Zur eisgekühlten Lösung von 5,8 g (3-(3,5-Dimethyl-thiomorpholin-1,1-dioxid-4-yl)-sydnonimin-hydrochlorid in 20 ml Wasser werden 3,9 g Natriumbicarbonat gegeben und anschließend die Lösung von 3 g Chlorameisensäureethylester in 20 ml Methylenchlorid zugefügt und die Mischung 6 Stunden im Eisbad gerührt. Die organische Phase wird abgetrennt, getrocknet und eingeengt. Der Rückstand wird säulenchromatografisch aufgearbeitet (Kieselgel, $CH_2Cl$ : MeOH = 97 : 3). Die entsprechenden Fraktionen werden eingeengt, der Rückstand mit Diethylether verrührt und abgesaugt.
Ausbeute : 2,1 g
Schmelzpunkt : 180°C (Zers.)

Beispiel 5

N-Benzoyl-3-(2,6-dimethylpiperidin-1-yl)-sydnonimin wird analog Beispiel 2 durch Umsetzung mit 3,2 g Benzoylchlorid statt 2,5 g Chlorameisensäure-ethylester hergestellt und aus Isopropanol/Petrolether umkristallisiert.
Ausbeute : 3,4 g
Schmelzpunkt : 141-142°C

Beispiel 6

N-Cyclohexylcarbonyl-3-(2,6-dimethylpiperidin-1-yl)-sydnonimin-hydrochlorid wird analog Beispiel 2 hergestellt durch Verwendung von 3,3 g Cyclohexancarbonsäurechlorid statt 2,5 g Chlorameisensäure-ethylester. Die ölige Base wird in Essigester gelöst und mit Chlorwasserstoff als Salz gefällt.
Ausbeute : 3,8 g
Schmelzpunkt : 111°C (Zers.)

Beispiel 7

N-(Allylthio)-acetyl-3-(2,6-dimethylpiperidin-1-yl)-sydnonimin-hydrochlorid wird analog Beispiel 6 hergestellt durch Verwendung von 3,4 g (Allylthio)-acetylchlorid statt 2,5 Chlorameisensäureethylester und aus Diisopropylether mit Chlorwasserstoff gefällt.
Ausbeute : 2,9 g
Schmelzpunkt : 91°C (Zers.)

Beispiel 8

N-n-Butoxy-acetyl-3-(2,6-dimethylpiperidin-1-yl)-sydnonimin-hydrochlorid wird analog Beispiel 7 durch Verwendung von 3,4 g n-Butoxy-acetylchlorid statt Chlorameisensäureethylester hergestellt und aus Diethylether mit HCl gefällt.
Ausbeute : 2,3 g
Schmelzpunkt : 101°C (Zers.)

Beispiel 9

N-Isopropoxycarbonyl-3-(2,6-dimethylpiperidin-1-yl)-sydnonimin wird analog Beispiel 2 hergestellt (2,7 g Chlorameisensäureisopropylester statt 2,5 g Chlorameisen säureethylester) und aus Isopropylester umkristallisiert.
Ausbeute : 4,0 g
Schmelzpunkt : 89-90°C

Beispiel 10

N-(2-Isopropoxy)-ethoxy-carbonyl-3-(2,6-dimethylpiperidin-1-yl)-sydnonimin wird analog Beispiel 2 hergestellt (3,0 g Chlorameisensäureisopropoxyethylester statt 2,5 g Chlorameisensäure-ethylester).
Ausbeute : 4,5 g
Schmelzpunkt : Öl

Beispiel 11

N-(Imidazol-1-yl-carbonyl)-3-(2,6-dimethylpiperidin-1-yl)-sydnonimin.

Die Mischung von 3,7 g 3-(2,6-Dimethylpiperidin-1-yl)-sydnonimin-hydrochlorid, 2,4 g Carbonyldiimidazol, 2,5 g Natriumbicarbonat und 50 ml Methylenchlorid wird 16 Stunden bei Raumtemperatur gerührt. Nach dem Abziehen des Lösungsmittels wird der Rückstand in 40 ml 2-N-Salzsäure aufgenommen und mit Diethylether ausgeschüttelt. Nach Neutralisation der wäßrigen Phase wird das Produkt mit Diethylether exrahiert, die Etherphase getrocknet und eingeengt. Der Rückstand wird aus Isopropylether umkristallisiert.
Ausbeute : 2,8 g
Schmelzpunkt : 119-120°C

Beispiel 12

3-(2,5-Dimethylpyyrolidin-1-yl)-sydnonimin-hydrochlorid.

Die Herstellung dieser Verbindung erfolgt analog Beispiel 1, ausgehend von 11,4 g N-(2,5-Dimethylpyyrolidino-aminoacetonitril und 7,6 g Natriumnitrit.
Ausbeute : 4,4 g
Schmelzpunkt : 120°C (Zers.)

Beispiel 13

N-Nitroso-3-(2,6-dimethylpiperidino)-sydnonimin.

Diese Verbindung wird durch Umsetzung von 3 g 3-(2,6-Dimethylpiperidino)-sydnonimin-hydrochlorid mit 0,93 g Natriumnitrit in Wasser erhalten, wobei soviel Salzsäure zugesetzt wird, daß der pH Wert zwischen 2 und 3 liegt.
Ausbeute : 2,1 g
Schmelzpunkt : 74°C (Zers.)

Analog zu Beispiel 2 werden die in der nachfolgenden Tabelle angegebenen Verbindungen der Formel Ib hergestellt.

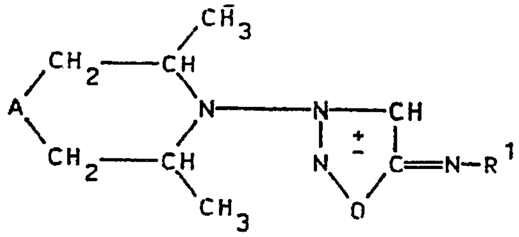

(Ib)

| Nr. | A | $R^1$ | Fp in $^oC$ |
|---|---|---|---|
| 14 | $-CH_2-$ | $-COCH_3$ | 115 (Zers.) |
| 15 | $-CH_2-$ | $-COCH(CH_3)_2$ | 131 (Zers.) |
| 16 | $-CH_2-$ | 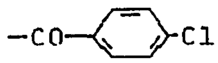 | 128 |
| 17 | $-CH_2-$ | 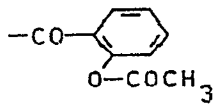 | 91 (Zers.) |
| 18 | - | 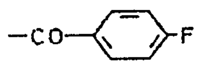 | 132 |
| 19 | - | 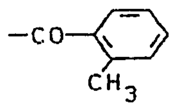 | 109 |
| 20 | - | 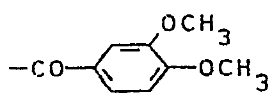 | 138 |
| 15 | | | |

| Nr. | A | $R^1$ | Fp in °C |
|---|---|---|---|
| 21 | $-CH_2-$ | 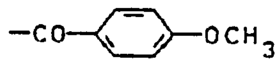 | 129 - 131 |
| 22 | $-CH_2-$ | 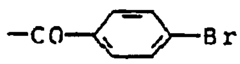 | 138 (Zers.) |
| 23 | $-CH_2-$ | 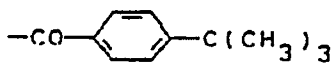 | 118 (Zers.) (Hydrochlorid) |
| 24 | $-CH_2-$ | 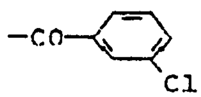 | 131 - 133 |
| 25 | $-CH_2-$ | 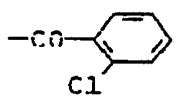 | 101 - 102 |
| 26 | $-CH_2-$ | 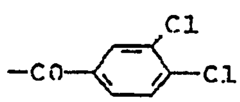 | 88 - 90 |
| 27 | $-CH_2-$ | 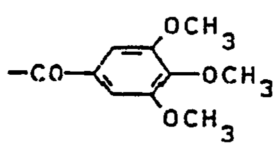 | 149 - 151 |
| 28 | $-CH_2-$ | 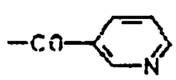 | 97 - 98 |
| 29 | $-CH_2-$ | 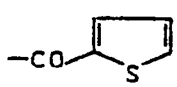 | 117 - 118 |
| 30 | $-CH_2-$ | 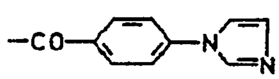 | 108 - 110 |

| Nr. | A | $R^1$ | Fp in °C |
|---|---|---|---|
| 31 | $-CH_2-$ | 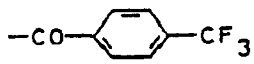 | 120 - 121 |
| 32 | $-CH_2-$ | 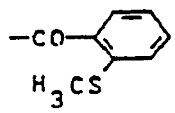 | 86 - 88 |
| 33 | $-CH_2-$ | 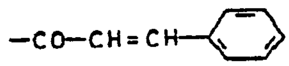 | 149 - 152 |
| 34 | $-CH_2-$ | 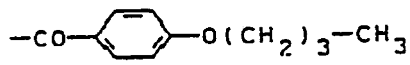 | 97 - 99 (Zers.) (Hydrochlorid) |
| 35 | $-CH_2-$ | $-CO_2(CH_2)_9-CH_3$ | 104 (Zers.) (Hydrochlorid) |
| 36 | $-CH_2-$ | $-CO_2(CH_2)_{17}-CH_3$ | 81 (Zers.) (Hydrochlorid) |

Die Angabe "–" für A bei den Tabellenbeispielen 18 bis 20 bedeutet eine direkte Bindung, d.h. bei den Beispielen 18 bis 20 ist in 3-Stellung des Sydnonimins ein 2,5-Di methylpyrrolidin-1-ylrest vorhanden.

In den nachfolgenden Beispielen A bis F werden pharmazeutische Präparate beschrieben.

## Beispiel A

Gelatineweichkapseln, enthaltend 5 mg Wirkstoff pro Kapsel :

| | pro Kapsel |
|---|---|
| Wirkstoff | 5 mg |
| Aus Kokosfett fraktioniertes Triglycerid-Gemisch | 150 mg |
| Kapselinhalt | 155 mg |

## Beispiel B

Injektionslösung, enthaltend 1 mg Wirkstoff pro ml :

| | | pro ml |
|---|---|---|
| Wirkstoff | | 1,0 mg |
| Polyethylenglycol 400 | | 0,3 ml |
| Natriumchlorid | | 2,7 mg |
| Wasser zu Injektionszwecken | ad | 1 ml |

Beispiel C

Emulsion, enthaltend 3 mg Wirkstoff pro 5 ml

| | | pro 100 ml Emulsion |
|---|---|---|
| Wirkstoff | | 0,06 g |
| Neutralöl | | q.s. |
| Natriumcarboxymethylcellulose | | 0,6 g |
| Polyoxyethylen-stearat | | q.s. |
| Glycerin rein | | 0,2 bis 2,0 g |
| Geschmacksstoff | | q.s. |
| Wasser (entsalzt oder destilliert) | ad | 100 ml |

Beispiel D

Rektale Arzneiform, enthaltend 4 mg Wirkstoff pro Suppositorium

| | | pro Suppositorium |
|---|---|---|
| Wirkstoff | | 4 mg |
| Suppositoriengrundmasse | ad | 2 g |

## Ansprüche

**Patentansprüche für die benannten Vertragstaaten : AT, BE, CH, DE, FR, GB, IT, LI, NL, SE**

1. Substituierte 3-Aminosydnonimine der allgemeinen Formel I

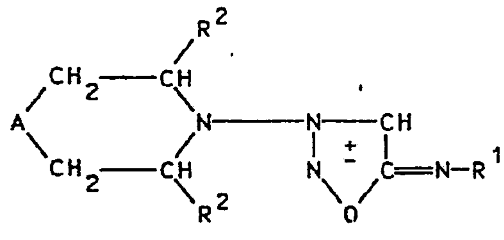

(I)

und ihre pharmakologisch annehmbaren Säureadditionssalze, worin
A den Rest $-CH_2-$, $-O-$, $-S(O_n)-$, $-N(R^3)-$ oder eine direkte Bindung,
$R^1$ Wasserstoff, NO oder den Rest $-COR^4$,

$R^2$, $R^3$ Alkyl mit 1 bis 4 C-Atomen oder Phenylalkyl mit 1 bis 4 C-Atomen im Alkylrest,

$R^4$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann ; einen cycloaliphatischen Rest mit 5 bis 7 C-Atomen ; einen bicycloaliphatischen Rest mit 7 bis 14 C-Atomen ; einen tricycloaliphatischen Rest mit 7 bis 16 C-Atomen ; einen Alkoxyrest mit 1 bis 18 C-Atomen, der auch durch Alkoxy mit 1 bis 6 C-Atomen substituiert sein kann ; einen Aryloxyrest mit 6 bis 10 C-Atomen ; einen Arylrest mit 6 bis 10 C-Atomen ; einen Arylrest mit 6 bis 10 C-Atomen, der mono-, di- oder trisubstituiert durch 1 bis 3 Halogenatome und/oder 1 bis 3 Alkylreste mit 1 bis 4 C-Atomen und/oder 1 bis 3 Alkoxyreste mit 1 bis 4 C-Atomen und/oder 1 oder 2 Nitrogruppen und/oder 1 oder 2 Hydroxygruppen und/oder 1 oder 2 Alkylcarbonyloxyreste mit 1 bis 4 C-Atomen und/oder 1 bis 3 Alkylthioreste mit 1 bis 4 C-Atomen und/oder einen Trifluormethylrest und/oder einen Imidazolylrest ist ; Imidazolyl ; Pyridyl ; Thienyl ; Styryl, n die Zahl 0, 1 oder 2, bedeuten.

2. Substituierte 3-Aminosydnonimine nach Anspruch 1, dadurch gekennzeichnet, daß $R^2$ Methyl bedeutet.

3. Substituierte 3-Aminosydnonimine nach Anspruch 1 und/oder 2, dadurch gekennzeichnet, daß A $-CH_2-$, eine direkte Bindung, $-S(O_2)-$ oder $-O-$ bedeutet.

4. 3-(2,6-Dimethylpiperidin-1-yl)-sydnonimin und seine pharmakologisch annehmbaren Säureadditionssalze.

5. 3-(2,6-Dimethylpiperidin-1-yl)-N-benzoyl-sydnonimin und seine pharmakologisch annehmbaren Säureadditionssalze.

6. 3-(2,6-Dimethylpiperidin-1-yl)-N-allylthio-methylcarbonyl-sydnonimin und seine pharmakologisch annehmbaren Säureadditionssalze.

7. 3-(2,6-Dimethylpiperidin-1-yl)-N-(p-anisoyl)-sydnonimin und seine pharmakologisch annehmbaren Säureadditionssalze.

8. Verfahren zur Herstellung der in einem oder mehreren der Ansprüche 1 bis 3 angegebenen Verbindungen der Formel I oder einer der in den Ansprüchen 4 bis 7 angegebenen Verbindung, durch gekennzeichnet, daß eine Verbindung der allgemeinen Formel II

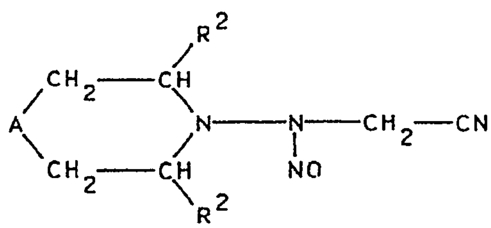

(II)

worin A und $R^2$ die in einem oder mehreren der Ansprüche 1 bis 3 angegebenen Bedeutungen besitzen, in einem Lösungs-, Dispergier- oder Verdünnungsmittel mit Hilfe eines Cyclisierungsmittels, das in wäßriger Lösung einen pH-Wert von 3 oder darunter einstellt, zu einer Verbindung der Formel Ia

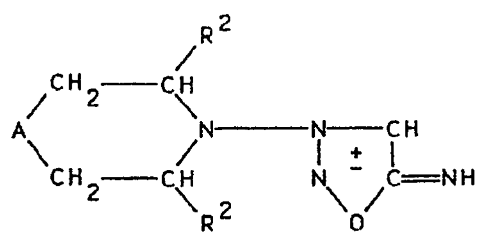

(Ia)

cyclisiert wird und daß diese oder ein Säureadditionssalz für den Fall, daß eine Verbindung der Formel I mit $R^1 = -COR^4$ hergestellt wird, mit einem Acylierungsmittel acyliert wird, das den Rest $-COR^4$ einführt, oder für den Fall, daß eine Verbindung der Formel I mit $R^1 = -NO$ hergestellt werden soll, nitrosiert wird und die erhaltene Verbindung gegebenenfalls in ein Säureadditionssalz überführt wird.

9. Verfahren nach Anspruch 8, dadurch gekennzeichnet, daß die Cyclisierung bei Temperaturen von −10 bis 40°C, vorzugsweise 0 bis 20°C, durchgeführt wird.

10. Substituierte 3-Amino-sydnonimine eines oder mehrerer der Ansprüche 1 bis 7 oder ihre pharmakologisch annehmbaren Säureadditionssalze als pharmakologische Wirkstoffe zur Anwendung bei der Bekämpfung und Vorbeugung cardiovaskulärer Erkrankungen.

11. Pharmazeutisches Präparat, dadurch gekennzeichnet, daß es eine Verbindung nach einem oder mehreren der Ansprüche 1 bis 7 und/oder ein Säureadditionssalz davon als Wirkstoff zusammen mit pharmazeutisch annehmbaren Träger-und Zusatzstoffen und gegebenenfalls noch eine oder mehrere andere pharmokologische Wirkstoffe enthält.

**Patentansprüche für die benannten Vertragsstaaten : ES, GR**

1. Verfahren zur Herstellung von substituierten 3-Aminosydnoniminen der allgemeinen Formel I

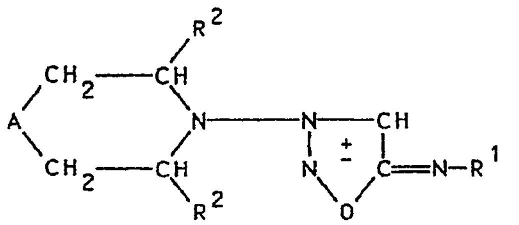

(I)

und ihrer pharmakologisch annehmbaren Säureadditionssalze, worin

A den Rest $-CH_2-$, $-O-$, $-S(O_n)-$, $-N(R^3)-$ oder eine direkte Bindung,

$R^1$ Wassestoff, NO oder den Rest $-COR^4$,

$R^2$, $R^3$ Alkyl mit 1 bis 4 C-Atomen oder Phenylalkyl mit 1 bis 4 C-Atomen im Alkylrest,

$R^4$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann ; einen cycloaliphatischen Rest mit 5 bis 7 C-Atomen ; einen bicyloaliphatischen Rest mit 7 bis 14 C-Atomen ; einen tricyloaliphatischen Rest mit 7 bis 16 C-Atomen ; einen Alkoxyrest mit 1 bis 6 C-Atomen, der auch durch Alkoxy mit 1 bis 6 C-Atomen substituiert sein kann ; einen Aryloxyrest mit 6 bis 10 C-Atomen ; einen Arylrest mit 6 bis 10 C-Atomen ; einen Arylrest mit 6 bis 10 C-Atomen ; der mono-, di- oder trisubstituiert durch 1 bis 3 Halogenatome und/oder 1 bis 3 Alkylreste mit 1 bis 3 C-Atome und/oder 1 oder 2 Nitrogruppen und/oder 1 oder 2 Hydroxygruppen und/oder 1 oder 2 Alkylcarbonyloxyreste mit 1 bis 4 C-Atomen ist ; Imidazolyl, n die Zahl 0, 1 oder 2, bedeuten, dadurch gekennzeichnet, daß eine Verbindung der allgemeinen Formel II

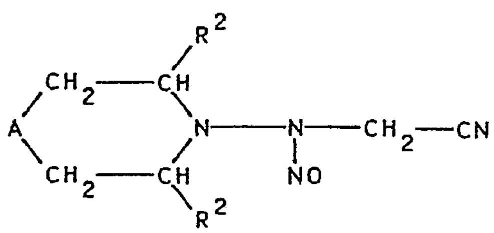

(II)

worin A und $R^2$ die angegbenen Bedeutungen besitzen, in einem Lösungs-, Dispergier- oder Verdünnungsmittel mit Hilfe eines Cyclisierungsmittels, das in wäßriger Lösung einen pH-Wert von 3 oder darunter einstellt, zu einer Verbindung der Formel Ia

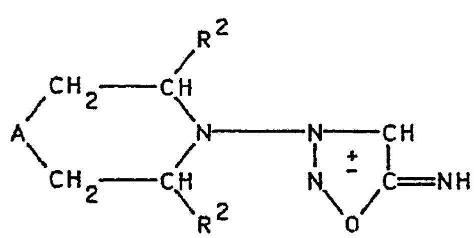

(Ia)

cyclisiert wird und daß diese oder ein Säureadditionssalz für deN Fall, daß eine Verbingung der Formel I mit $R^1 = -COR^4$ hergestellt wird, mit einem Acylierungsmittel acyliert wird, das den Rest $-COR^4$ einführt oder für den Fall, daß eine Verbindung der Formel I mit $R^1 = -NO$ hergestedllt werden soll, nitrosiert wird in ein erhaltene Verbindung gegebenenfalls in ein Säureadditionsalz überführt wird.

2. Verfahren nach Anspruch 1, dadurch gekennzeichnet, daß die Cyclisierung bei Temperaturen von −10

bis 40°C, vorzugsweise 0 bis 20°C, durchgeführt wird.

3. Verfahren nach Anspruch 1 und/oder 2, dadurch gekennzeichnet, daß eine Verbindung der Formel II oder Ia mit $R^2$ Methyl eingesetzt wird.

4. Verfahren nach Anspruch 1 und/oder 2, dadurch gekennzeichnet, daß eine Verbindung der Formel II oder Ia mit A $-CH_2-$, einer direkten Bindung, $-S(O_2)-$ oder $-O-$ eingesetzt wird.

5. Verfahren nach Anspruch 1 und/oder 2, dadurch gekennzeichnet, daß eine Verbindung der Formel II oder Ia mit $R^2$ Methyl und A $-CH_2-$ eingesetzt wird.

6. Verfahren nach Anspruch 1 und/oder 2, dadurch gekennzeichnet, daß eine Verbindung der Formel II oder Ia mit $R^2$ Methyl und A $-CH_2-$ eingesetzt und die entstehende Verbindung mit einem Acylierungsmittel acyliert wird, das den Benzoylrest einführt.

7. Verfahren nach Anspruch 1 und/oder 2, dadurch gekennzeichnet, daß eine Verbindung der Formel II oder Ia mit $R^2$ Methyl und A $-CH_2-$ eingesetzt und die entstehende Verbidung mit einem Acylierungsmittel acyliert wird, das den Allylthio-methyl-carbonyl-rest einführt.

8. Verfahren nach Ansprush 1 und/oder 2, dadurch gekennzeichnet, daß eine Verbindung der Formel II oder Ia mit $R^2$ Methyl und A $-CH_2-$ eingesetzt und die entstehende Verbindung mit einem Acylierungsmittel acyliert wird, das den p-Anisoylrest einführt.

9. Verwendung substituierter 3-Aminosydnonimine der allgemeinen Formel I

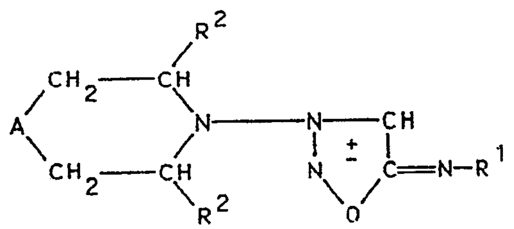

(I)

und ihrer pharmakologisch annehmbaren Säureadditionssalze, worin

A den Rest $-CH_2-$, $-O-$, $-S(O_n)-$, $-N(R^3)-$ oder eine direkte Bindung,

$R^1$ Wasserstoff, NO oder den Rest $-COR^4$,

$R^2$, $R^3$ Alkyl mit 1 bis 4 C-Atomen oder Phenylalkyl mit 1 bis 4 C-Atomen im Alkylrest,

$R^4$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann ; einen cycloaliphatischen Rest mit 5 bis 7 C-Atomen ; einen bicycloaliphatischen Rest mit 7 bis 14 C-Atomen ; einen tricycloaliphatischen Rest mit 7 bis 16 C-Atomen ; einen Alkoxyrest mit 1 bis 6 C-Atomen, der auch durch Alkoxy mit 1 bis 6 C-Atomen substituiert sein kann ; einen Aryloxyrest mit 6 bis 10 C-Atomen ; einen Alkoxycarbonylrest mit insgesamt 2 bis 7 C-Atomen ; einen Arylrest mit 6 bis 10 C-Atomen ; einen durch 1 bis 3 Halogenatome und/oder 1 bis 3 C-Alkylreste mit 1 bis 3 C-Atomen oder 1 oder 2 Nitrogruppen und/oder 1 oder 2 Hydroxygrupopen und/oder 1 oder 2 Alkylcarbonyloxyreste mit 1 bis 4 C-Atomen mono-, di- oder trisubstituierten Arylrest mit 6 bis 10 C-Atomen ; Imidazolyl ; n die Zahl 0, 1 oder 2, bedeuten, als pharmakologische Wirkstoffe zur Herstellung pharmazeutischer Präparate, die der Bekämpfung und Vorbeugung cardiovaskulärer Erkrankungen dienen.

## Claims

### Claims for the Contracting States : AT, BE, CH, DE, FR, GB, IT, LI, NL, SE

1. Substituted 3-aminosydnonimines of the general formula I

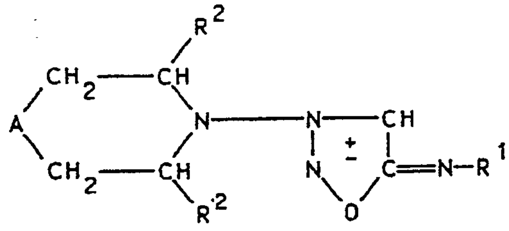

(I)

and the pharmaologically acceptable acid addition salts thereof, in which

A denotes the radical $-CH_2-$, $-O-$, $-S(O_n)-$, $-N(R^3)-$ or a direct bond,

$R^1$ denotes hydrogen, NO or the radical $-COR^4$,

$R^2$ and $R^3$ denote alkyl having 1 to 4 C atoms or phenylalkyl having 1 to 4 C atoms in the alkyl radical, $R^4$ denotes an aliphatic radical which has 1 to 6 C atoms and can also be substituted by alkoxy having 1 to 6 C atoms or an aliphatic thio radical having up to 4 C atoms ; a cycloaliphatic radical having 5 to 7 C atoms ; a bicycloaliphatic radical having 7 to 14 C atoms ; a tricycloaliphatic radical having 7 to 16 C atoms ; an alkoxy radical which as 1 to 18 C atoms which can also be substituted by alkoxy having 1 to 6 C atoms ; an aryloxy radical having 6 to 10 C atoms ; an alkoxycarbonyl radical having a total of 2 to 7 C atoms ; an aryl radical having 6 to 10 C atoms which is mono-, di- or trisubstituted by 1 to 3 halogen atoms and/or 1 to 3 alkyl radicals having 1 to 4 C atoms and/or 1 to 3 alkoxy radicals having 1 to 4 C atoms and/or 1 or 2 nitro groups and/or 1 or 2 hydroxy groups and/or 1 or 2 alkylcarbonyloxy groups having 1 to 4 C atoms and/or a trifluoromethyl radical and/or an imidazolyl radical ; imidazolyl ; pyridyl ; thienyl ; styryl, n denotes the number 0, 1 or 2.

2. Substituted 3-aminosydnonimines according to Claim 1, characterized in that $R^2$ denotes methyl.

3. Substituted 3-aminosydnonimines according to Claim 1 and/or 2, characterized in that A denotes $-CH_2-$, a direct bond, $-S(O_2)-$ or $-O-$.

4. 3-(2,6-Dimethylpiperidin-1-yl)-sydnonimine and the pharmacologically acceptable acid addition salts thereof.

5. 3-(2,6-Dimethylpiperidin-1-yl)-N-benzoylsydnonimine and the pharmacologically acceptable acid addition salts thereof.

6. 3-(2,6-Dimethylpiperidin-1-yl)-N-allylthiomethylcarbonyl-sydnonimine and the pharmacologically acceptable acid addition salts thereof.

7. 3-(2,6-Dimethylpiperidin-1-yl)-N-(p-anisoyl)-sydnonimine and the pharmacologically acceptable acid addition salts thereof.

8. Process for preparing the compound of formula I indicated in one or several of Claims 1 to 3 or a compound indicated in Claims 4 to 7 characterized in that a compound of the general formula II

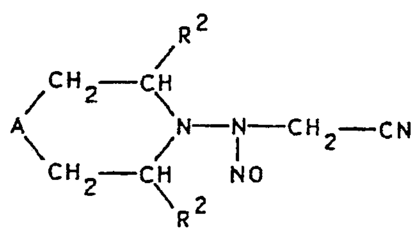

(II)

wherein A and $R^2$ have the meanings indicated in one or several of Claims 1 to 3 is cyclized, in a solvent, dispersant, or diluent, by means of a cyclizing agent establishing in aqueous solution a pH of 3 or below, to give a compound of the formula Ia

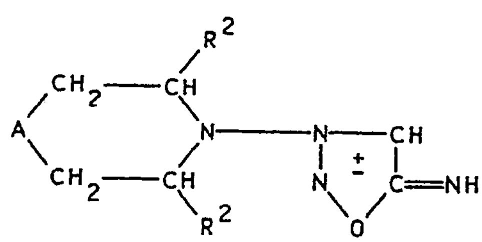

(Ia)

and that this compound or an acid addition salt, in case a compound of the formula I wherein $R^1$ denotes $-COR^4$ is prepared, is acylated with an acylating agent introducing the radical $-COR^4$ or, in case a compound of the formula I wherein $R^1$ denotes $-NO$ is prepared, is nitrosated and the compound obtained is optionally converted into an acid addition salt.

9. The process of Claim 8, characterized in that the cyclization reaction is carried out at temperatures between −10 and 40°C, preferably 0 to 20°C.

10. Substituted 3-aminosydnonimines of one or several of Claims 1 to 7, or pharmacologically acceptable acid addition salts thereof as pharmacological active ingredients for use in combating and preventing cardiovascular diseases.

11. Pharmaceutical preparation characterized in that it contains a compound of one or several of Claims

1 to 7 and/or an acid addition salt thereof as active ingredient together with pharmaceutically acceptable excipients and additives and optionally one or more other pharmacological active ingredients.

**Claims for the Contracting States : ES, GR**

1. Process for preparing substituted 3-aminosydnonimines of the general formula I

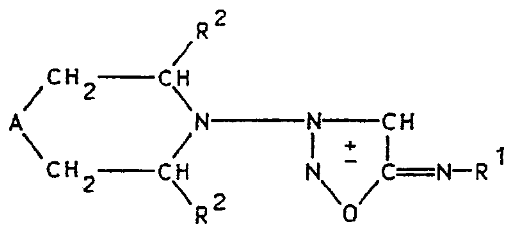

(I)

and the pharmaologically acceptable acid addition salts thereof, in which

A denotes the radical $-CH_2-$, $-O-$, $-S(O_n)-$, $-N(R^3)-$ or a direct bond,

$R^1$ denotes hydrogen, NO or the radical $-COR^4$,

$R^2$ and $R^3$ denote alkyl having 1 to 4 C atoms or phenylalkyl having 1 to 4 C atoms in the alkyl radical, $R^4$ denotes an aliphatic radical which has 1 to 6 C atoms and can also be substituted by alkoxy having 1 to 6 C atoms or an aliphatic thio radical having up to 4 C atoms ; a cycloaliphatic radical having 5 to 7 C atoms ; a bicycloaliphatic radical having 7 to 14 C atoms ; a tricycloaliphatic radical having 7 to 16 C atoms ; an alkoxy radical which has 1 to 6 C atoms which can also be substituted by alkoxy having 1 to 6 C atoms ; an aryloxy radical having 6 to 10 C atoms ; in alkoxycarbonyl radical having a total of 1 to 7 C atoms ; an aryl radical having 6 to 10 C atoms which is mono-, di- or trisubstituted by 1 to 3 halogen atoms and/or 1 to 3 alkyl radicals having 1 to 3 C atoms and/or 1 or 2 nitro groups and/or 1 or 2 hydroxy groups and/or 1 or 2 alkylcarbonyloxy groups having 1 to 4 C atoms ; imidazolyl ; n denotes the number 0, 1 or 2, characterized in that a compound of the general formula II

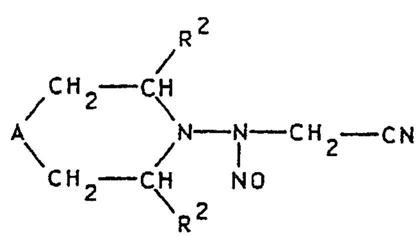

(II)

wherein A and $R^2$ have the meanings indicated is cyclized, in a solvent, dispersant, or diluent, by means of a cyclizing agent establishing in aqueous solution a pH of 3 or below, to give a compound of the formula Ia

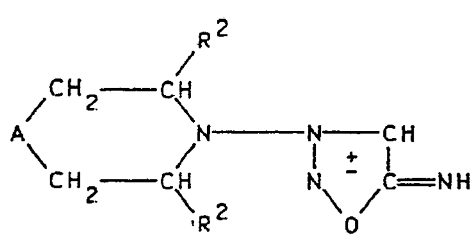

(Ia)

and that this compound or an acid addition salt, in case a compound of the formula I wherein $R^1$ denotes $-COR^4$ is prepared, is acylated with an acylating agent introducing the radical $-COR^4$ or, in case a compound of the formula I wherein $R^1$ denotes $-NO$ is prepared, is nitrosated and the compound obtained is optionally converted into an acid addition salt.

2. Process according to Claim 1, characterized in that the cyclization is carried out at temperatures between −10 to 40°C, preferably 0 to 20°C.

3. Process according to Claim 1 and/or 2, characterized in that a compound of formula II or Ia wherein $R^2$

denotes methyl is used.

4. Process according to Claim 1 and/or 2, characterized in that a compound of formula II or Ia wherein A denotes $-CH_2-$, a direct bond, $-S(O_2)-$ or $-O-$ is used.

5. Process according to Claim 1 and/or 2, characterized in that a compound of formula II or Ia wherein $R^2$ denotes methyl and A denotes $-CH_2-$ is used.

6. Process according to Claim 1 and/or 2, characterized in that a compound of formula II or Ia wherein $R^2$ denotes methyl and A denotes $-CH_2-$ is used and the resulting compound is acylated with an acylating agent introducing the benzoyl radical.

7. Process according to Claim 1 and/or 2, characterized in that a compound of formula II or Ia wherein $R^2$ denotes methyl and A denotes $-CH_2-$ is used and the resulting compound is acylated with an acylating agent introducing the allylthio-methyl-carbonyl radical.

8. Process according to Claim 1 and/or 2, characterized in that a compound of formula II or Ia wherein $R^2$ denotes methyl and A denotes $-CH_2-$ is used and the resulting compound is acylated with an acylating agent introducing the p-anisoyl radical.

9. The use of substituted 3-aminosydnonimines of the general formula I

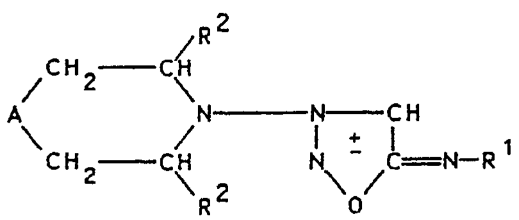

( I )

and the pharmaologically acceptable acid addition salts thereof, in which

A denotes the radical $-CH_2-$, $-O-$, $-S(O_n)-$, $-N(R^3)-$ or a direct bond,

$R^1$ denotes hydrogen, NO or the radical $-COR^4$,

$R^2$ and $R^3$ denote alkyl having 1 to 4 C atoms or phenylalkyl having 1 to 4 C atoms in the alkyl radical, $R^4$ denotes an aliphatic radical which has 1 to 6 C atoms and can also be substituted by alkoxy having 1 to 6 C atoms or an aliphatic thio radical having up to 4 C atoms ; a cycloaliphatic radical having 5 to 7 C atoms ; a bicycloaliphatic radical having 7 to 14 C atoms ; a tricycloaliphatic radical having 7 to 16 C atoms ; an alkoxy radical which has 1 to 6 C atoms which can also be substituted by,alkoxy having 1 to 6 C atoms ; an aryloxy radical having 6 to 10 C atoms ; an alkoxycarbonyl radical having a total of 2 to 7 C atoms ; an aryl radical having 6 to 10 C atoms which is mono-, di- or trisubstituted by 1 to 3 halogen atoms and/or 1 to 3 C alkyl radicals having 1 to 3 C atoms and/or 1 or 2 nitro groups and/or 1 or 2 hydroxy groups and/or 1 or 2 alkylcarbonyloxy groups having 1 to 4 C atoms ; imidazolyl ; n denotes the number 0, 1 or 2, as pharmacological active ingredients for preparing pharmaceutical preparations serving to combat and prevent cardiovascular diseases.

## Revendications

### Revendications pour les Etats contractants : AT, BE, CH, DE, FR, GB, IT, LI, NL, SE

1. 3-Aminosydnonimines substituées de formule générale I ci-dessous :

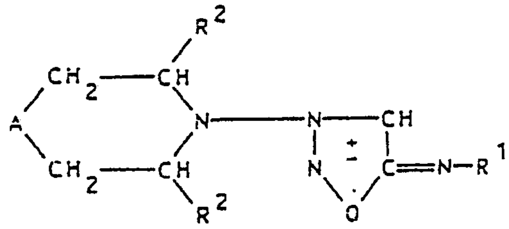

(I)

ainsi que leurs sels d'addition d'acides utilisables en pharmacologie, formule dans laquelle

A représente le radical $-CH_2-$, $-O-$, $-S(O_n)-$, $-N(R^3)-$ ou une liaison directe,

$R^1$ l'hydrogène, le groupe nitroso (-NO) ou un radical $-COR^4$,

$R^2$ et $R^3$ sont des alkyles en $C_1$-$C_4$ ou des phenylalkyles à alkyle en $C_1$-$C_4$,

$R^4$ représente un radical aliphatique en $C_1$-$C_6$ pouvant être substitué par un alcoxy en $C_1$-$C_6$ ou par un radical thioaliphatique pouvant avoir jusqu'à 4 atomes de carbone ; un radical cycloaliphatique en $C_5$-$C_7$ ; un radical bicycloaliphatique en $C_7$-$C_{14}$ ; un radical tricycloaliphatique en $C_7$-$C_{16}$ ; un alcoxy en $C_1$-$C_{18}$ pouvant être également substitué par un alcoxy en $C_1$-$C_6$ ; un aryloxy en $C_6$-$C_{10}$ ; un alcoxycarbonyle ayant au total de 2 à 7 atomes de carbone ; un aryle en $C_6$-$C_{10}$ sans substituants ou ayant de 1 à 3 substituants, à savoir de 1 à 3 atomes d'halogènes et/ou de 1 à 3 alkyles en $C_1$-$C_4$ et/ou de 1 à 3 alcoxy en $C_1$-$C_4$ et/ou 1 ou 2 groupes nitro et/ou 1 ou 2 hydroxyles et/ou 1 ou 2 alkylcarbonyloxy en $C_1$-$C_4$ et/ou de 1 à 3 alkylthio en $C_1$-$C_4$ et/ou un radical trifluorométhyle et/ou un radical imidazolyle ; ou encore un radical imidazolyle, pyridyle, thiényle ou styryle, et n est le nombre 0, 1 ou 2.

2. 3-Aminosydnonimines selon la revendication 1 dans lesquelles $R^2$ est le groupe méthyle.

3. 3-Aminosydnonimines selon la revendication 1 et/ou 2 dans lesquelles A est le groupe $-CH_2-$, une liaison directe ou bien le radical $-S(O_n)-$ ou $-O-$.

4. 3-(2,6-Diméthylpipéridin-1-yl)-sydnonimine et ses sels d'addition d'acides utilisables en pharmacologie.

5. 3-(2,6-Diméthylpipéridin-1-yl)-N-benzoyl-sydnonimine et ses sels d'addition d'acides utilisables en pharmacologie.

6. 3-(2,6-Diméthylpipéridin-1-yl)-N-allylthio-méthylcarbonyl-sydnonimine et ses sels d'addition d'acides utilisales en pharmacologie.

7. 3-(2,6-Diméthylpipéridin-1-yl)-N-(p-anisoyl)-sydnonimine et ses sels d'addition d'acides utilisables en pharmacologie.

8. Procédé de préparation des composés selon les revendicatiors 1 à 7, procédé caractérisé en ce que l'on cyclise un composé de formule générale II

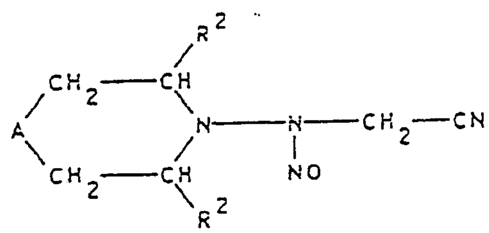

(II)

A et $R^2$ ayant les significations qui ont été données à une ou plusieurs des revendications 1 à 3, dans un solvant, un dispersant ou un diluant, avec un agent de cyclisation qui donne en solution aqueuse un pH de 3 ou moins, en un composé de formule générale Ia

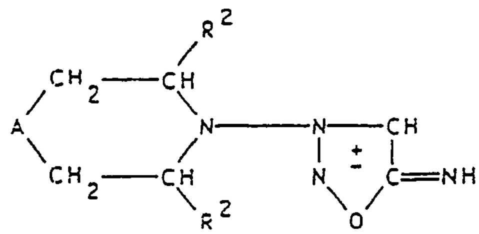

(Ia)

et on acyle celui-ci ou son sel d'addition d'acide, si l'on veut obtenir un composé de formule I dans lequel $R^1$ est le groupe $-COR^4$, avec un agent d'acylation fixant ce groupe $-COR^4$, ou bien, si l'on veut obtenir un composé de formule I dans lequel $R^1$ est le groupe $-NO$, on nitrose ce composé Ia, et le cas échéant on transforme le composé ainsi contenu en un sel d'addition d'acide.

9. Procédé selon la revendication 8, caractérisé en ce que la cyclisation se fait à des températures de −10 à 40°C, de préférence de 0 à 20°C.

10. 3-Amino-sydnonimines selon les revendications 1 à 7 ou leurs sels d'addition d'acides utilisables en pharmacologie, comme matières actives pour combattre ou prévenir des maladies cardio-vasculaires.

11. Préparations pharmaceutiques caractérisées en ce qu'elles comprennent des composés selon les revendications 1 à 7 et/ou des sels d'addition d'acide de ces composés comme matière active, avec des véhicules et additifs pour usages pharmaceutiques et le cas échéant également une ou plusieurs autres matières ayant une activité pharmacologique.

## Revendications pour les Etats contractants : ES, GR

1. Procédé de préparation de 3-aminosydnonimines substituées de formule générale I ci-dessous :

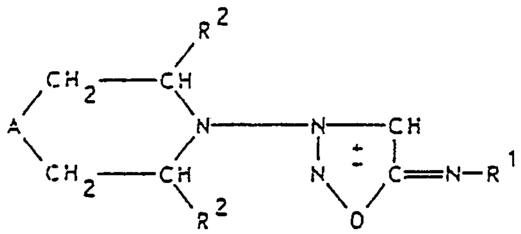

(I)

et de leurs sels d'addition d'acides utilisables en pharmacologie, formule dans laquelle

A représente le radical $-CH_2-$, -O-, $-S(O_n)-$, $-N(R^3)-$ ou une liaison directe,

$R^1$ l'hydrogène, le groupe nitroso (-NO) ou un radical $-COR^4$,

$R^2$ et $R^3$ sont des alkyles en $C_1$-$C_4$ ou des phénylalkyles à alkyle en $C_1$-$C_4$,

$R^4$ représente un radical aliphatique en $C_1$-$C_6$ pouvant être substitué par un alcoxy en $C_1$-$C_6$ ou par un radical thioaliphatique pouvant avoir jusqu'à 4 atomes de carbone ; un radical cycloaliphatique en $C_5$-$C_7$ ; un radical bicycloaliphatique en $C_7$-$C_{14}$ ; un radical tricycloaliphatique en $C_7$-$C_{16}$ ; un alcoxy en $C_1$-$C_{16}$ pouvant être également substitué par un alcoxy en $C_1$-$C_8$ ; un aryloxy en $C_6$-$C_{10}$ ; un alcoxycarbonyle ayant au total de 2 à 7 atomes de carbone ; un aryle en $C_6$-$C_{10}$ sans substituants ou ayant de 1 à 3 substituants, à savoir de 1 à 3 atomes d'halogènes et/ou de 1 à 3 alkyles en $C_1$-$C_3$ et/ou 1 ou 2 groupes nitro et/ou 1 ou 2 hydroxyles et/ou 1 ou 2 alkylcarbonyloxy en $C_1$-$C_4$ ; ou encore un radical imidazolyle ; et n est le nombre 0, 1 ou 2, procédé caractérisé en ce que l'on cyclise un composé de formule générale II

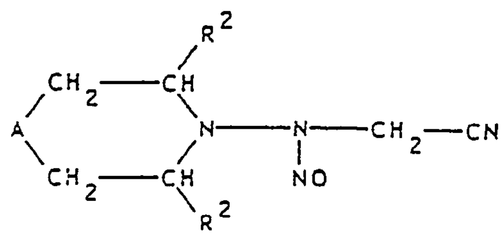

(II)

A et $R^2$ ayant les significations indiquées, dans un solvant, un dispersant ou un diluant, avec un agent de cyclisation qui donne en solution aqueuse un pH de 3 ou moins, en un composé de formule générale Ia

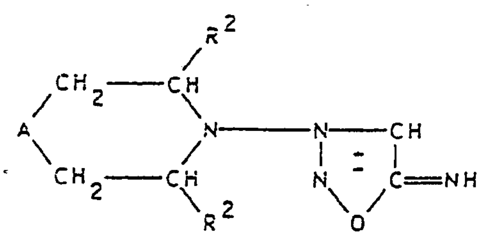

(Ia)

et on acyle celui-ci ou son sel d'addition d'acide, si l'on veut obtenir un composé de formule I dans lequel $R^1$ est le groupe $-COR^4$, avec un agent d'acylation fixant ce groupe $-COR^4$, ou bien, si l'on veut obtenir un composé de formule I dans lequel $R^1$ est le groupe -NO, on nitrose ce composé Ia, et le cas échéant on transforme le composé ainsi obtenu en un sel d'addition d'acide.

2. Procédé selon la revendication 1, caractérisé en ce que la cyclisation se fait à des températures de -10 à 40°C, de préférence de 0 à 20°C.

3. Procédé selon la revendication 1 et/ou 2, caractérisé en ce que dans le composé de formule II ou Ia $R^2$ est le groupe méthyle.

4. Procédé selon la revendication 1 et/ou 2, caractérisé en ce que dans le composé de formule II ou Ia A est le groupe $-CH_2-$, une liaison directe ou bien le radical $-S(O_2)-$ ou -O-.

5. Procédé selon la revendication 1 et/ou 2, caractérisé en ce que dans le composé de formule II ou Ia $R^2$

est le groupe méthyle et A le groupe $-CH_2-$.

6. Procédé selon la revendication 1 et/ou 2, caractérisé en ce que dans le composé de formule II ou la $R^2$ est le groupe méthyle et A le groupe $-CH_2-$, et en ce que l'on acyle le composé formé avec un agent d'acylation qui axe le radical benzoyle.

7. Procédé selon la revendication 1 et/ou 2, caractérisé en ce que dans le composé de formule II ou la $R^2$ est le groupe méthyle et A le groupe $-CH_2-$, et en ce que l'on acyle le composé formé avec un agent d'acylation qui fixe le radical allylthio-méthyl-carbonyle.

8. Procédé selon la revendication 1 et/ou 2, caractérisé en ce que dans le composé de formule II ou la $R^2$ est le groupe méthyle et A le groupe $-CH_2-$, et en ce que l'on acyle le composé formé avec un agent d'acylation qui fixe le radical p-anisoyle.

9. L'emploi des 3-aminosydnonimines substituées de formule générale I

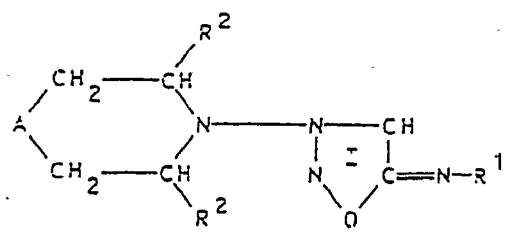

(I)

ainsi que de leurs sels d'addition d'acides utilisables en pharmacologie, formule dans laquelle

A représente le radical $-CH_2-$, $-O-$, $-S(O_n)-$, $-N(R^3)-$ ou une liaison directe,

$R^1$ l'hydrogène, le groupe nitroso (–NO) ou un radical $-COR^4$,

$R^2$ et $R^3$ sont des alkyles en $C_1$-$C_4$ ou des phénylalkyles à alkyle en $C_1$-$C_4$,

$R^4$ représente un radical aliphatique en $C_1$-$C_6$ pouvant être substitué par un alcoxy en $C_1$-$C_6$ ou par un radical thioaliphatique pouvant avoir jusqu'à 4 atomes de carbone ; un radical cycloaliphatique en $C_5$-$C_7$ ; un radical bicycloaliphatique en $C_7$-$C_{14}$ ; un radical tricycloaliphatique en $C_7$-$C_{16}$ ; un alcoxy en $C_1$-$C_{18}$ pouvant être également substitué par un alcoxy en $C_1$-$C_6$ ; un aryloxy en $C_6$-$C_{10}$ ; un alcoxycarbonyle ayant au total de 2 à 7 atomes de carbone ; un aryle en $C_6$-$C_{10}$ sans substituants ou ayant de 1 à 3 substituants, à savoir de 1 à 3 atomes d'halogènes et/ou de 1 à 3 alkyles en $C_1$-$C_3$ et/ou 1 ou 2 groupes nitro et/ou 1 ou 2 hydroxyles et/ou 1 ou 2 alkylcarbonyloxy en $C_1$-$C_4$ ; ou encore un radical imidazolyle ; ou encore $R^4$ est un radical imidazolyle, et n est le nombre 0, 1 ou 2,

comme matières actives pour la fabrication de médicaments ou préparations pharmaceutiques destinés à combattre et à prévenir des maladies cardio-vasculaires.